Europäisches Patentamt

(19) European Patent Office

Office européen des brevets

(11) Publication number: **0 052 087**
**A1**

(12) # EUROPEAN PATENT APPLICATION

(21) Application number: **81830218.4**

(22) Date of filing: **10.11.81**

(51) Int. Cl.³: **A 61 N 1/36, A 61 N 1/08**

(30) Priority: **12.11.80 IT 958480**

(43) Date of publication of application: **19.05.82**
**Bulletin 82/20**

(84) Designated Contracting States: **BE CH DE FR GB LI NL SE**

(71) Applicant: **Sodi, Fiorello, Via Ugo Foscolo 5, I-50018 Scandicci Firenze (IT)**
Applicant: **Talluri, Antonio, Via Montepilli 8, I-50012 Bagno a Ripoli Firenze (IT)**

(72) Inventor: **Sodi, Fiorello, Via Ugo Foscolo 5, I-50018 Scandicci Firenze (IT)**
Inventor: **Talluri, Antonio, Via Montepilli 8, I-50012 Bagno a Ripoli Firenze (IT)**

(74) Representative: **Mannucci, Gianfranco, Ufficio Tecnico Ing. A. Mannucci Via della Scala 4, I-50123 Firenze (IT)**

(54) **Process and apparatus for the correction of scoliosis and other spinal deformities.**

(57)    In order to correct the scoliosis and similar spine deformities, muscular stimuli are generated aiming to provoke a spine deflection in the direction opposite to the one taken up by the spine itself as a consequence of the disease, by the application – on the skin – of two electrodes suitably spaced apart which receive pulsing signals capable of stimulating the muscular fascia; a gradual attenuation of the single electrical stimuli of the muscular fascia is provided as well as an initial gradual increase of the stimuli intensity at the beginning of each daily treatment.

EP 0 052 087 A1

ACTORUM AG

COMPLETE DOCUMENT

## DESCRIPTION

The invention refers to an apparatus and a process as well for the correction of the scoliosis or other spine malformations.

The process is already known, as well as an equipment, for carrying out muscular stimuli aiming to provoke a spine deflection in the direction opposite to the one taken up by the spine itself as a consequence of the disease. The muscular stimulus is obtained by the application - on the skin - of two transcutaneous electrodes, suitably spaced apart, which receive pulsing signals able to stimulate the muscular fascia. The signals are mostly formed with the aid of a carrier wave having a frequency appropriate for the modulation of said carrier wave to obtain signals able to provoke periodic muscular contractions, avoiding the muscular fatigue and the subsequent "tetanus" which would occur in case of continuous stimulus. Whereas the carrier wave may have a frequency of, for instance, 25 Hz, the stimuli for the muscular tissue are made up of trains of pulses which may have for example a duration in the range of six seconds with an equivalent period of pulse interruption. The modulation necessary to obtain the trains of pulses may be performed by a slow oscillator, while at present,

- 2 -

said modulation is carried out by an initial inclined ramp to get some graduality in the muscle stimulus so as to cause the muscle contraction to occur gradually; besides nowadays, the stimuli stop suddenly.

The known apparatus and methods are intended for providing applications at night during the sleeping hours and it is customary that on the early days the applications are carried out by starting with slighter stimuli to make the muscles accustomed to these prolonged intermittent stimuli which have the purpose to strain the spine in the direction suitable to correct the malformation.

The apparatus and methods known at present are improved according to the invention with the purpose to make the treatment more effective and to reduce the trouble phenomena which the currently used treatments may provoke in the subjects being treated. A further object of the apparatus and improved methods according to the invention is to obtain a more complete treatment and hence, a more satisfying correction of the deformations coming from the scoliosis.

According to the invention, there is provided a gradual attenuation of the single electrical stimuli of the muscular mass so as to avoid the intervention of natural corrective stimuli from the brain, which

- 3 -

take place in the present systems at the end of each 0052087
periodic stimuli during the treatment.

To obtain this, there may be used a suitable pro
grammable generator and there may be provided means
to regulate the time of each ramp of attenuation of
the stimuli supplied to the electrodes for the purposes
mentioned above.

A method and an apparatus improved according to
the invention provide also an initial gradual increase
of the intensity of the electrical stimuli supplied
through the electrodes to the or each muscular fascia,
at the beginning of each treatment for intermittent
stimulus, that is, for example, at the beginning of
each daily treatment during the night sleeping hours.
Through this, the musculature is gradually "warmed up"
and the nervous system gets painlessly accustomed to
the electrical stimulus, whereby the body accepts more
comfortably the treatment stimuli as there is excluded
any sudden start of them as it occurs instead with the
current methodology.

This gradual increase may have a length from six
to ten minutes; there has been found that the intensity
of the first stimuli may be, anyhow, of an intermediate
magnitude respect to the final value of same stimuli;
for example the starting stimuli may be of 30 milliamperes
and the normal running stimuli in the range of 50 to 70
milliamperes.

- 4 -

Means may be provided to control the duration of this initial "warming up" of the musculature, that is, of this graduality of the stimulus increase at the start of each treatment, and this time of treatment for the "warming up" may be for example, in the range of eight to thirty minutes, especially in the range of ten to fifteen minutes at the beginning of each treatment.

An improved apparatus according to the invention includes, advantageously, more than a pair of cooperating electrodes, to obtain the simultaneous corrective treatment by stimulating a plurality of muscular fasciae at various positions, said fasciae able to receive (simultaneous or out of phase) stimuli in relation to the opportunity of stimulating - in a simultaneous or out of phase way - the muscular fasciae affected by the two or more pairs of used electrodes.

An improved apparatus according to the invention can also include, advantageously, an alarm or signalling system able to operate at the moment an electrode gets accidentally detached from the zone of the skin where it has been applied - by means of adhesive plasters or other - to effect the treatment. Practically, there may be provided means for determining the above mentioned alarm signalling, when the resistance between the two electrodes of a pair increases beyond a certain limit,

- 5 -

which occurs in case of removal, that is, detaching of an electrode. This may be obtained with the use, for example, of a trigger.

Means may also be provided for determining an analogous alarm signal, when a drop of the electrical stimuli power takes place below a programmed threshold, for example, below 30 milliamperes.

The accompanying drawing shows an exemplification - not limitative - of the criteria accomplishment on which the invention is based. In particular:

Fig.1 shows a diagram of both initial and normal running stimuli;

Fig.2 shows a signal as obtained by a carrier wave modulation, of course with altered dimensions for the sake of the drawing evidence;

Fig.3 shows in a very schematic way a possible application of the pairs of electrodes for correcting two opposite deflections; and

Fig.4 shows a circuit diagram.

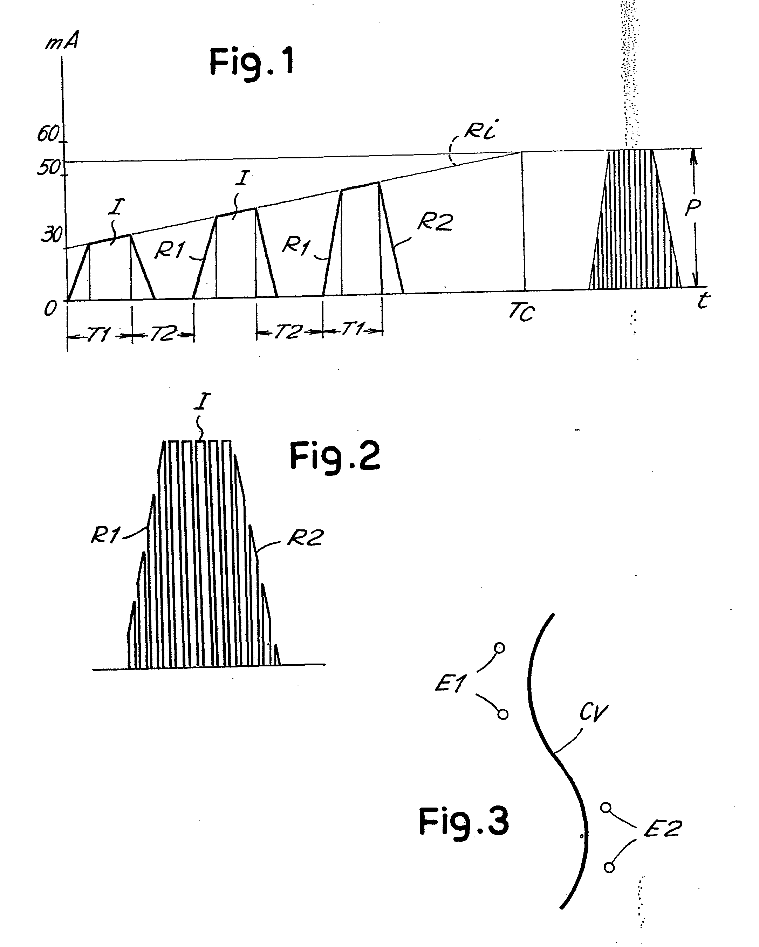

According to the accompanying drawing, in Fig.1 there is shown a diagram of the stimuli in amplitude in function of the time. The stimuli have a medium length T1 in the range of six seconds, with time intervals T2, between them, equal to or different from T1. Each pulse I, as shown also in Fig.2 has an initial ramp R1 which makes the muscular contraction to occur gradually; each pulse I is further defined by an end ramp R2 also inclined, to get a gradual attenuation of the signal and hence of the muscular contraction, so as to make the stimulus physiologically more acceptable, without tiring out the subject.

In Fig.1 a diagram is shown illustrating an initial period up to the time Tc during which a gradual increase of the pulses intensity takes place according to a ramp Ri rising from a value of the signal intensity which may be about half the running one, said running being reached after a time Tc ranging from six to ten minutes. This graduality is imposed at the beginning of each daily or anyway periodical treatment. During subsequent treatments it is possible to change the power P of the pulses to get the most suitable one for each subject. The graduality according to the ramp Ri of the stimuli accomplished at the beginning of each treatment, in order to warm. up the musculature, may be constant with the changing of the power P, or may be quite an almost direct function of this power.

In Fig.3 there is shown in a very schematic way a manner of utilizing two pairs of electrodes E1 and E2 to correct, through muscular stimuli, the two deflections which may occur in some cases in the spine CV as a consequence of the kind of scoliosis so called with multiple deflections. The two pairs of electrodes are so located as to operate on the muscular fasciae which are on the side of the respective convexities in order to operate as to attenuate the same convexities by means of the muscular contractions. The presence of two pairs of electrodes permits to carry on the treatment simultaneously on the two deflections. In this way there are obtained corrective treatments of the double side deformations, and by appropriate positions of the electrodes it is also possible to obtain corrections of deflections on the lateral plane.

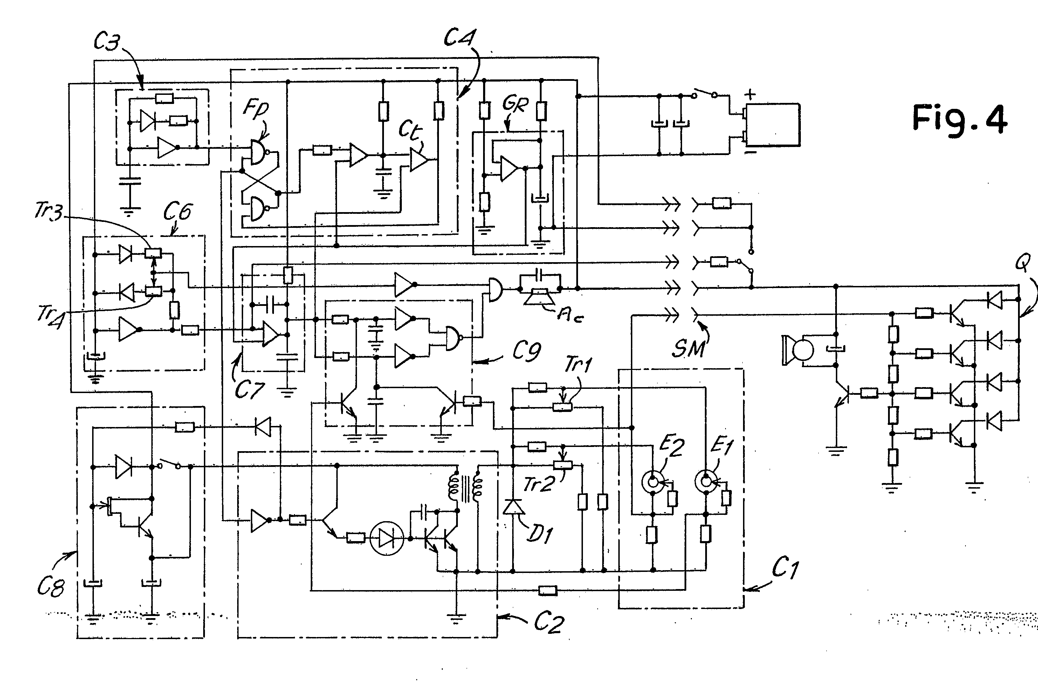

In Fig.4 there is shown a circuit diagram for carrying out the invention; the circuit can feed the two pairs of electrodes E1 and E2 placed on the user circuit C1. In the diagram the block C2 is the power amplifier circuit, down stream of which a diode D1 provides to shut out reverse stimuli, while the trimmers Tr1, Tr2 are provided for regulating the voltages and hence the power P. The circuit C3 is a frequency generator for the carrier wave, for example at 25 Hz. A programmable generator C4, controlled through a flip-flop Fp forming part of the generator itself, permits the formation of the initial ramps Ri of pulses I. A phase voltage comparator Ct is a part of this programmed generator circuit C4; the pulsing signal is supplied through the flip-flop Fp. By GR there is indicated a reference voltage generator. A circuit C6 forms a slow oscillator to obtain pulses I at the frequency, for example, of six seconds pulse and six seconds interval; it comprises trimmers Tr3 and Tr4 for controlling the times of stimulus and rest (ON and OFF) respectively. A block C7 with a ramp integrator is used for controlling the time of the ramps R1 and R2. The circuit C8 is a generator of initial preheating to regulate the time Tc of ramp Ri which provides for the gradual increase of the pulses intensity during the initial period of every treatment for the warming up of the muscular fascia (or fasciae) **and** for avoiding the initial troubles which would occur with stimuli taking place from **the very** beginning of the running condition. By C9 there is indicated an alarm circuit with controllers for the alarm threshold. By Ac there is indicated an acoustic warner, fed according to the frequency of the slow oscillator C6, through a signal adaptor Ad and under the control of circuit C9.

The operator has the possibility to regulate the running intensity P of     signal I.

By means of a loose cable connected through a multiple plug SM it is possible to have a remote manual drive for controls, X-ray photographs and the like. A switch SW3 at rest shuts out the pulses neutralizing circuit C7. The circuit C7 is put at rest by the resistor R37. By commuting on SW3 we can have, at will, a continuous stimulus, for example for X-ray photographs or for getting extended pulses for various observations (useful to the physician).

**On the remote** control knob, there may be, advanta geously, a visual display Q with some LEDs which indicate some threshold values of the stimulating pulse intensity. There may be also provided an acoustic signal Z2 to indicate a particular value taken up by the pulse and suited e.g. for a X-ray photographic exposure.

It must be understood that the drawing shows only one embodiment of the invention which may vary as regard to forms and dispositions without, nevertheless, departing from the basic concept of the invention itself.

# C L A I M S

1) A process for correcting scoliosis and other malformations involving the generation of muscular stimuli aiming to urge a spine deflection in the direction opposite to the one taken up by the spine itself as a consequence of the disease, by the application of two electrodes suitably spaced apart which receive pulsing signals capable of repeatedly stimulating the muscular mass, characterized by the fact that there is provided a gradual attenuation of the single electrical stimuli of the muscular mass, so as to avoid the intervention of natural corrective stimuli from the brain and the consequent trouble.

2) A process according to the preceding claim, characterized by the fact that it provides an initial gradual increase of the stimuli intensity at the beginning of each treatment, especially daily treatment.

3) Apparatus to carry out the process of the preceding claims, characterized by the fact that it comprises a programmable generator and means for generating a ramp of final attenuation of each stimulus supplied to the electrodes.

4) Apparatus according to claim 3, characterized by the fact that it comprises means able to determine an initial gradual increase of the amplitude of the electrical stimuli supplied through the electrodes to the or each muscular fascia, at the beginning of each treatment with intermittent stimuli, that is, e.g., at the beginning of each treatment during the night sleeping hours.

5) Apparatus according to the preceding claims, characterized by the fact that it comprises more than one pair of cooperating electrodes, to obtain the simultaneous corrective treatment by stimulating a plurality of muscular fasciae in various positions.

6) Apparatus according to the preceding claims, characterized by the fact that it comprises a signalling system - especially an alarm system - able to operate the moment an electrode has accidentally come off from the skin.

7) Apparatus according to the preceding claims, characterized by the fact that it comprises means for producing the alarm warning above mentioned when the absorption between the electrodes of a same pair drops down beyond a certain limit (programmed threshold alarm).

8) Apparatus according to the preceding claims, characterized by the fact that it comprises a remote manual control, connectable for neutralizing, at will, the automatic control of the oscillator, and for obtai-

- 12 -

ning pulses of voluntary duration.

9) Apparatus according to claim 8, characterized by the fact that, to the remote manual control, a system is associated for controlling the intensity of the pulses.

10) A process and an apparatus for the correction of scoliosis; all as described and illustrated by way of example in the accompanying drawing.

1/2

0052087

Fig.1

Fig.2

Fig.3

Fig. 4

0052087

European Patent
Office

EUROPEAN SEARCH REPORT

Application number

EP 81 83 0218

## DOCUMENTS CONSIDERED TO BE RELEVANT

| Category | Citation of document with indication, where appropriate, of relevant passages | Relevant to claim |
|---|---|---|
| Y | FR - A - 2 319 385 (MEDTRONIC)<br><br>* Page 5, lines 12-25; page 6, lines 6-24 * | 1,5,8-10 |
| Y | MEDICAL AND BIOLOGICAL ENGINEERING AND COMPUTING, vol. 15, January 1977 STEVENAGE (GB) V. STANIC et al.: "Effect of gradually modulated electrical stimulation on the plasticity of artificially evoked movements" pages 62-66<br><br>* Page 63, right-hand column, paragraph 2; page 64, figure 4 * | 1,3 |
| A | FR - A - 2 079 780 (MARTINIE)<br><br>* Page 1, lines 4-16 * | 1 |
| A | US - A - 4 177 819 (KOFSKY)<br><br>* Column 1, lines 32-34; column 2, lines 28-68; column 3, lines 51-66 * | 1-4, 6,7 |
| A | US - A - 3 645 267 (MEDTRONIC)<br><br>* Column 2, lines 30-52 * | 1,2,4 |
| A | FR - A - 2 398 509 (MOSKOVSKY OBLASTNOI)<br><br>* Page 13, line 8 to page 15, line 4 * | 8,9 |
| E | EP - A - 0 033 747 (MATSUSHITA)<br><br>* Page 3, line 32 to page 4, line 32; page 5, lines 2-10 * | 1-4,6, 7 |

The present search report has been drawn up for all claims

| Place of search | Date of completion of the search | Examiner |
|---|---|---|
| The Hague | 18-02-1982 | SIMON |

EPO Form 1503.1 06.78

**CLASSIFICATION OF THE APPLICATION (Int. Cl. 3)**

A 61 N    1/36
          1/08

**TECHNICAL FIELDS SEARCHED (Int.Cl. 3)**

A 61 N    1/36
          1/08

**CATEGORY OF CITED DOCUMENTS**

X: particularly relevant if taken alone
Y: particularly relevant if combined with another document of the same category
A: technological background
O: non-written disclosure
P: intermediate document
T: theory or principle underlying the invention
E: earlier patent document, but published on, or after the filing date
D: document cited in the application
L: document cited for other reasons

&: member of the same patent family, corresponding document